(19) Europäisches Patentamt
European Patent Office
Office européen des brevets

(11) EP 2 723 451 B1

# (12) EUROPEAN PATENT SPECIFICATION

(45) Date of publication and mention of the grant of the patent:
**14.02.2018 Bulletin 2018/07**

(21) Application number: **12730900.3**

(22) Date of filing: **22.06.2012**

(51) Int Cl.:
*A61Q 5/10* (2006.01) *A61K 8/19* (2006.01)
*A61K 8/22* (2006.01) *A61K 8/27* (2006.01)
*A61K 8/36* (2006.01) *A61K 8/41* (2006.01)
*A61K 8/49* (2006.01)

(86) International application number:
**PCT/EP2012/062104**

(87) International publication number:
**WO 2012/175684 (27.12.2012 Gazette 2012/52)**

## (54) HAIR DYEING PROCESS EMPLOYING AT LEAST ONE DIHYDROXYFLAVANOL, A MANGANESE OR ZINC SALT, HYDROGEN PEROXIDE, (BI)CARBONATE, AN ALKALINE AGENT AND A MAGNESIUM, MOLYBDENUM OR CALCIUM SALT

HAARFÄRBEVERFAHREN MIT MINDESTENS EINEM DIHYDROXYFLAVANOL, EINEM MANGAN- ODER ZINKSALZ, WASSERSTOFFPEROXID, (BI)CARBONAT, EINEM ALKALISCHEN MITTEL UND EINEM MAGNESIUM-, MOLYBDÄN- ODER CALCIUMSALZ

PROCÉDÉ DE COLORATION CAPILLAIRE UTILISANT AU MOINS UN DIHYDROXYFLAVONOL, UN SEL DE MANGANÈSE OU DE ZINC, DU PEROXYDE D'HYDROGÈNE, UN (BI)CARBONATE, UN AGENT ALCALIN ET UN SEL DE MAGNÉSIUM, DE MOLYBDÈNE OU DE CALCIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2011 FR 1155525**
**26.09.2011 US 201161539103 P**

(43) Date of publication of application:
**30.04.2014 Bulletin 2014/18**

(73) Proprietor: **L'Oréal**
**75008 Paris (FR)**

(72) Inventors:
- **RONDOT, Christophe**
  **F-77290 Mitry-Mory (FR)**
- **CHOISY, Patrick**
  **F-37270 Montlouis-sur-Loire (FR)**

(74) Representative: **Casalonga**
**Casalonga & Partners**
**Bayerstraße 71/73**
**80335 München (DE)**

(56) References cited:
**EP-A2- 2 196 180** **EP-A2- 2 196 181**
**US-A1- 2007 251 024** **US-A1- 2009 249 563**

- **DWECK A C: "Natural ingredients for colouring and styling", INTERNATIONAL JOURNAL OF COSMETIC SCIENCE, KLUWER ACADEMIC PUBLISHERS, DORDRECHT, NL, vol. 24, 1 January 2002 (2002-01-01), pages 287-302, XP002500910, ISSN: 0142-5463, DOI: 10.1046/J.1467-2494.2002.00148.X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 723 451 B1

## Description

The present invention relates to a process for dyeing keratin fibres, in particular human keratin fibres such as the hair, in which said fibres are treated using one or more cosmetic compositions comprising a) one or more dihydroxyflavanol derivative(s), b) one or more manganese or zinc salt(s), c) hydrogen peroxide or one or more hydrogen peroxide-generating system(s), d) one or more (bi)carbonate(s) or one or more (bi)carbonate-generating system(s), e) one or more alkalinizing agent(s) other than the (bi)carbonate(s) and f) one or more metal salt(s) chosen from magnesium, molybdenum and calcium salts, it being understood that the ingredient f) is applied together, i.e. as co-treatment, with at least one of the other ingredients a) to e).

It is known practice to obtain "permanent" colourations with dyeing compositions containing oxidation dye precursors, which are generally known as oxidation bases, such as ortho- or para-phenylenediamines, ortho- or para-aminophenols and heterocyclic compounds. These oxidation bases are colourless or weakly coloured compounds, which, when combined with oxidizing products, may give rise to coloured compounds by a process of oxidative condensation. It is also known that the shades obtained may be varied by combining these oxidation bases with couplers or colouration modifiers, the latter being chosen especially from aromatic meta-diamines, meta-aminophenols, meta-diphenols and certain heterocyclic compounds such as indole compounds. This oxidation dyeing process consists in applying, to the keratin fibres, bases or a mixture of bases and couplers with hydrogen peroxide ($H_2O_2$ or aqueous hydrogen peroxide solution), as oxidizing agent, in leaving to diffuse, and in then rinsing the fibres. The colourations resulting therefrom are permanent, strong and resistant to external agents, especially to light, bad weather, washing, perspiration and rubbing.

However, the commercial hair colourings which contain them generally have the drawback of staining the clothing, of causing odour and comfort problems, and of damaging the keratin fibres. This is particularly the case with oxidation dyes.

However, the colourations obtained are not strong enough, and are not very chromatic, in particular in the case of hair fibres.

Moreover, it is known practice to use metals at acid pH for dyeing keratin fibres in amounts similar to those employed for dyes using a mordanting process, which consists in preparing the fibres before carrying out the dyeing in order to obtain persistent colours (see, for example, Ullmann's Encyclopedia, "Metal and Dyes", 2005, § 5.1, p.8).

However, this process generally has the drawback of not always respecting the cosmetic appearance of the keratin fibre. Furthermore, the metals used, such as iron, result in neutral shades that are not very chromatic. Generally, such metals do not make it possible to obtain chromatic shades.

As examples. EP 2 196 180 and EP 2 196 181 relate to dyeing processes wherein a composition comprising orthodiphenol derivatives and metallic salts chosen from manganese and zinc salts is applied to keratin fibres. However, the coloration thus obtained is not sufficient.

There thus exists a real need to develop dyeing processes which make it possible to obtain strong, persistent and/or chromatic colourations using dihydroxyflavanols and which are less aggressive to keratin fibres. In particular, there exists a need to obtain colourations which satisfactorily withstand external agents (light, bad weather, shampooing) and which are persistent and homogeneous, i.e. which have a weak colouration selectivity between the root and the tip, while remaining strong and/or chromatic.

This aim is achieved by the present invention, a subject-matter of which is a process for dyeing keratin fibres, in particular human keratin fibres such as the hair, in which:

a first step consists in applying to said keratin fibres a composition comprising the following ingredients:

a) one or more dihydroxyflavanol derivative(s), represented by compounds of following formula (**I**) and also organic or inorganic acid salts thereof, optical, geometric and tautomeric isomers thereof, and solvates thereof such as the hydrates:

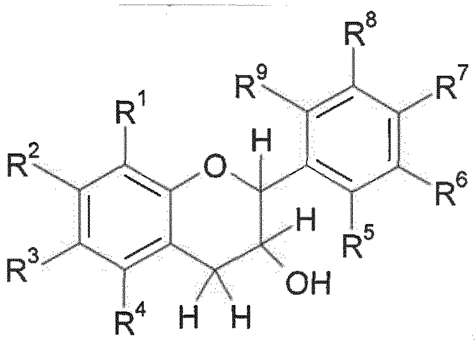

(I)

in which:

- $R^1$, $R^2$, $R^3$, $R^4$, $R^5$, $R^6$, $R^7$, $R^8$ and $R^9$, which are identical or different, represent:

  - a hydrogen atom,
  - a halogen atom.
  - a hydroxyl group,
  - a $(C_1$-$C_6)$alkyl group,
  - a $(C_1$-$C_6)$alkoxy group.
  - a $(C_1$-$C_6)$alkylthio group,
  - a carboxyl group,

    - an alkoxycarbonyl group,
    - an optionally substituted amino group,
    - an optionally substituted linear or branched alkenyl group,
    - an optionally substituted cycloalkyl group,
    - an aryl group,
    - a substituted aryl group,
    - a group containing one or more silicon atoms.
    - a (di)((hydroxy)$(C_1$-$C_6)$alkyl)amino group.
    - an -O-sugar group, such as -O-glucoside.

  - an R-Z-C(X)-Y-group with R representing a hydrogen atom or a $(C_1$-$C_6)$alkyl group, Y and Z, which may be identical or different, representing a bond, an oxygen or sulfur atom or an -N(R')- group with R' representing a hydrogen atom or a $(C_1$-$C_6)$alkyl group, Y possibly also representing a $(C_1$-$C_6)$alkylene group, and X representing an oxygen or sulfur atom, or N-R" with R" representing a hydrogen atom or a $(C_1$-$C_6)$alkyl group;
    it being understood that at least two adjacent radicals chosen from $R^1$, $R^2$, $R^3$, $R^4$, $R^5$, $R^6$, $R^7$. $R^8$ and $R^9$ represent a hydroxyl group,

b) one or more manganese salt(s) or one or more zinc salt(s),
c) hydrogen peroxide or one or more hydrogen peroxide-generating system(s), and
f) one or more metal salt(s) chosen from magnesium, molybdenum and calcium salts:

and then, a second step consists in applying to said keratin fibres a composition comprising the following ingredients:

d) one or more (bi)carbonate(s) or one or more (bi)carbonate-generating system(s), and
e) one or more alkalinizing agent(s) other than the bicarbonate(s);

it being understood that:

- at least one of the compositions used during said dyeing process has an alkaline pH, i.e. greater than 7, and
- the composition comprising the ingredient a) is aqueous.

Another subject-matter of the present invention is a cosmetic composition for the dyeing of keratin fibres,

comprising:

a) one or more dihydroxyflavanol derivative(s) as mentioned previously,
b) one or more manganese salt(s) or one or more zinc salt(s),
c) hydrogen peroxide or one or more hydrogen peroxide-generating system(s),
d) one or more (bi)carbonate(s) or one or more (bi)carbonate-generating system(s),
e) one or more alkalinizing agent(s) other than the bicarbonate(s), and
f) one or more metal salt(s) chosen from magnesium, molybdenum and calcium salts,

it being understood that the pH of the composition is alkaline, i.e. greater than 7, and preferably between 8 and 12. It is in particular between 8 and 10.5.

The cosmetic composition is aqueous.

The process according to the invention has the advantage of dyeing human keratin fibres, with persistent and, surprisingly, chromatic dyeing results. In particular, the dyeing process according to the invention makes it possible to produce colourations that are resistant to washing, perspiration, sebum and light without detrimentally modifying the fibres. Furthermore, the dyeing process employed makes it possible to induce a satisfactory "uptake" of the colouration.

Thus, another subject-matter of the invention is the use of one or more metal salt(s) f) for improving the chromaticity of keratin fibres, in particular human keratin fibres such as the hair, using one or more dihydroxyflavanol derivative(s) as previously defined.

Other subject-matters, characteristics, aspects and advantages of the present invention will emerge even more clearly on reading the description and the examples that follow.

For the purposes of the present invention, and unless otherwise indicated:

The "saturated or unsaturated, optionally fused rings" may also be optionally substituted.

The "alkyl" radicals are saturated, linear or branched, generally $C_1$-$C_{20}$, particularly $C_1$-$C_{10}$, hydrocarbon radicals, preferably $C_1$-$C_6$ alkyl radicals, such as methyl, ethyl, propyl, butyl, pentyl and hexyl.

The "alkenyl" radicals are unsaturated, linear or branched, $C_2$-$C_{20}$ hydrocarbon radicals; preferably comprising at least one double bond, such as ethylene, propylene, butylene, pentylene, 2-methylpropylene and decylene.

The "aryl" radicals are monocyclic or fused or nonfused polycyclic carbon-based radicals, preferentially comprising from 6 to 30 carbon atoms, at least one ring of which is aromatic; the choice is preferably made, from the aryl radical, of a phenyl, biphenyl, naphthyl, indenyl, anthracenyl and tetrahydronaphthyl.

The "alkoxy" radicals are alkyl-oxy radicals with alkyl as defined previously, preferably a $C_1$-$C_{10}$ alkyl, such as methoxy, ethoxy, propoxy and butoxy.

The "alkylthio" radicals are alkyl-S- radicals with alkyl as defined previously, preferably a $C_1$-$C_{10}$ alkyl, such as methylthio, ethylthio, propylthio and butylthio.

The "alkoxyalkyl" radicals are preferably ($C_1$-$C_{20}$)alkoxy($C_1$-C2o)alkyl radicals, such as methoxymethyl, ethoxymethyl, methoxyethyl, ethoxyethyl, etc.

The "cycloalkyl" radicals are generally $C_4$-$C_8$ cycloalkyl radicals, preferably the cyclopentyl and cyclohexyl radicals. The cycloalkyl radicals may be substituted cycloalkyl radicals, in particular substituted by alkyl, alkoxy, carboxylic acid, hydroxyl, amine and ketone groups.

The "alkyl" or "alkenyl" radicals, when they are "optionally substituted", may be substituted by at least one substituent carried by at least one carbon atom, chosen from:

- a halogen atom;
- a hydroxyl group;
- a $C_1$-$C_2$ alkoxy radical;
- a $C_1$-$C_{10}$ alkoxycarbonyl radical;
- a $C_2$-$C_4$ (poly)hydroxyalkoxy radical;
- an amino radical;
- a 5- or 6-membered heterocycloalkyl radical;
- an optionally cationic 5- or 6-membered heteroaryl radical, preferentially imidazolium, optionally substituted by a ($C_1$-$C_4$)alkyl radical, preferentially methyl;
- an amino radical substituted by one or two identical or different $C_1$-$C_6$ alkyl radicals, optionally carrying at least:

  * one hydroxyl group,
  * one amino group optionally substituted by one or two optionally substituted $C_1$-$C_3$ alkyl radicals, said alkyl radicals possibly forming, with the nitrogen atom to which they are attached, a saturated or unsaturated 5- to

7-membered heterocycle which is optionally substituted and which optionally comprises at least one other heteroatom identical to or different from nitrogen,
* a quaternary ammonium group $-N^+R'R''R'''$ $M^-$ for which R', R'' and R''', which may be identical or different, represent a hydrogen atom or a $C_1$-$C_4$ alkyl group; and $M^-$ represents the counterion of the corresponding organic acid, inorganic acid or halide,
* or an optionally cationic 5- or 6-membered heteroaryl radical, preferentially imidazolium, optionally substituted by a $(C_1$-$C_4)$alkyl radical, preferentially methyl;

- an acylamino (-NR-COR') radical in which the R radical is a hydrogen atom or a $C_1$-$C_4$ alkyl radical optionally carrying at least one hydroxyl group and the R' radical is a $C_1$-$C_2$ alkyl radical; a carbamoyl ($(R)_2N$-CO-) radical in which the R radicals, which may be identical or different, represent a hydrogen atom or a $C_1$-$C_4$ alkyl radical optionally carrying at least one hydroxyl group; an alkylsulfonylamino ($R'SO_2$-NR-) radical in which the R radical represents a hydrogen atom or a $C_1$-$C_4$ alkyl radical optionally carrying at least one hydroxyl group and the R' radical represents a $C_1$-$C_4$ alkyl radical or a phenyl radical; an aminosulfonyl ($(R)_2N$-$SO_2$-) radical in which the R radicals, which may be identical or different, represent a hydrogen atom or a $C_1$-$C_4$ alkyl radical optionally carrying at least one hydroxyl group;
- a carboxylic radical in acid or salified (preferably with an alkali metal or a substituted or unsubstituted ammonium) form;
- a cyano group;
- a nitro group;
- a carboxyl or glycosylcarbonyl group;
- a phenylcarbonyloxy group optionally substituted by one or more hydroxyl groups;
- a glycosyloxy group; and
- a phenyl group optionally substituted by one or more hydroxyl groups.

The term "carboxylates" is understood to mean carboxylic acid salt.
The term "carboxylic acid" is understood to mean a compound comprising at least one carboxylic acid -C(O)-OH group, preferably between 1 and 4 carboxylic acid groups, such as 1 or 2. More particularly, the carboxylic acid is chosen from: i) $(C_1$-$C_{10})$alkyl-$[C(O)$-$OH]_n$ and ii) het-$[C(O)$-$OH]_n$, with n an integer between 1 and 4 inclusive, preferably between 1 and 2, het representing a heterocyclic group, such as pyrrolidone, it being possible for the alkyl or het group to be optionally substituted by one or more groups chosen in particular from OH, and (di)$(C_1$-$C_6)$(alkyl)amino.
The "aryl" or "heterocyclic" radicals or the aryl or heterocyclic part of the radicals, when they are optionally substituted, may be substituted by at least one substituent carried by at least one carbon atom, chosen from:

- a $C_1$-$C_{10}$, preferably $C_1$-$C_8$, alkyl radical optionally substituted by one or more radicals chosen from the hydroxyl radical, $C_1$-$C_2$ alkoxy radical, (poly)hydroxy$(C_2$-$C_4)$alkoxy radical, acylamino radical, amino radical substituted by two identical or different $C_1$-$C_4$ alkyl radicals optionally carrying at least one hydroxyl group, or the two radicals possibly forming, with the nitrogen atom to which they are attached, a saturated or unsaturated and optionally substituted 5-to 7-membered, preferably 5- or 6-membered, heterocycle optionally comprising another heteroatom identical to or different from nitrogen;
- a halogen atom;
- a hydroxyl group;
- a $C_1$-$C_2$ alkoxy radical;
- a $C_1$-$C_{10}$ alkoxycarbonyl radical;
- a (poly)hydroxy$(C_2$-$C_4)$alkoxy radical;
- an amino radical;
- a 5- or 6-membered heterocycloalkyl radical;
- an optionally cationic 5- or 6-membered heteroaryl radical, preferentially imidazolium, optionally substituted by a $(C_1$-$C_4)$alkyl radical, preferentially methyl;
- an amino radical substituted by one or two identical or different $C_1$-$C_6$ alkyl radicals, optionally carrying at least:

* one hydroxyl group,
* one amino group optionally substituted by one or two optionally substituted $C_1$-$C_3$ alkyl radicals, said alkyl radicals possibly forming, with the nitrogen atom to which they are attached, a saturated or unsaturated and optionally substituted 5- to 7-membered heterocycle optionally comprising at least one other heteroatom identical to or different from nitrogen,
* one quaternary ammonium group $-N^+R'R''R'''$ $M^-$ for which R', R'' and R''', which may be identical or different, represent a hydrogen atom or a $C_1$-$C_4$ alkyl group; and $M^-$ represents the counterion of the corresponding organic acid, inorganic acid or halide,
* or one optionally cationic 5- or 6-membered heteroaryl radical, preferentially imidazolium, optionally substituted

by a ($C_1$-C4)alkyl radical, preferentially methyl;

- an acylamino (-NR-COR') radical in which the R radical is a hydrogen atom or a $C_1$-$C_4$ alkyl radical optionally carrying at least one hydroxyl group and the R' radical is a $C_1$-$C_2$ alkyl radical; a carbamoyl ($(R)_2N$-CO-) radical in which the R radicals, which may be identical or different, represent a hydrogen atom or a $C_1$-$C_4$ alkyl radical optionally carrying at least one hydroxyl group; an alkylsulfonylamino ($R'SO_2$-NR-) radical in which the R radical represents a hydrogen atom or a $C_1$-$C_4$ alkyl radical optionally carrying at least one hydroxyl group and the R' radical represents a $C_1$-$C_4$ alkyl radical or a phenyl radical; an aminosulfonyl ($(R)_2N$-$SO_2$-) radical in which the R radicals, which may be identical or different, represent a hydrogen atom or a $C_1$-$C_4$ alkyl radical optionally carrying at least one hydroxyl group,
- a carboxylic radical in acid or salified (preferably with an alkali metal or a substituted or unsubstituted ammonium) form;
- a cyano group;
- a nitro group;
- a polyhaloalkyl group, preferentially trifluoromethyl;
- a carboxyl or glycosylcarbonyl group;
- a phenylcarbonyloxy group optionally substituted by one or more hydroxyl groups;
- a glycosyloxy group; and
- a phenyl group optionally substituted by one or more hydroxyl groups.

The radicals "containing one or more silicon atoms" are preferably polydimethylsiloxane, polydiphenylsiloxane, polydimethylphenylsiloxane or stearoxydimethicone radicals.

The "heterocyclic" radicals are generally radicals comprising, in at least one ring, one or more heteroatoms chosen from O, N and S, preferably O or N, optionally substituted by in particular one or more alkyl, alkoxy, carboxylic acid, hydroxyl, amine or ketone groups. These rings may contain one or more oxo groups on the carbon atoms of the heterocycle.

Among the "heterocyclic" radicals that may be used, mention may be made of the furyl, pyranyl, pyrrolyl, imidazolyl, pyrazolyl, pyridyl and thienyl groups.

More preferably, the "heterocyclic" groups are fused groups, such as benzofuryl, chromenyl, xanthenyl, indolyl, isoindolyl, quinolyl, isoquinolyl, chromanyl, isochromanyl, indolinyl, isoindolinyl, coumarinyl or isocoumarinyl groups, these groups possibly being substituted, in particular by one or more OH groups.

The term "heterosides" or "glycosides" is understood to mean any compound resulting from the condensation of monosaccharides and non-glucide substances, also referred to as aglycones or genins.

The term "glycosyl radical" means a radical derived from a monosaccharide or monosaccharide.

a) <u>dihydroxyflavanol derivative</u>

In accordance with the present invention, the dyeing process employs one or more dihydroxyflavanol derivative(s) of formula (I) as previously defined.

A specific form of the invention relates to dihydroxyflavanol derivatives or mixtures of compounds.

According to one embodiment, $R^6$ and $R^7$ represent a hydroxyl group.

Preferably, $R^1$, $R^3$, $R^5$, $R^8$and $R^9$ represent hydrogen atoms and $R^2$ and $R^4$ represent a hydroxyl group.

The dihydroxyflavanol derivatives employed during the dyeing process according to the invention can be natural or synthetic.

Among the natural dihydroxyflavanol derivatives are compounds that may be present in nature and that are reproduced by chemical (semi)synthesis. The salts of the dihydroxyflavanol derivatives of the invention may be salts of acids or bases. The acids may be inorganic or organic.

Preferably, the acid is hydrochloric acid, which results in chlorides.

The bases may be inorganic or organic. In particular, the bases are alkali metal hydroxides, such as sodium hydroxide, which results in sodium salts.

According to a particular embodiment of the invention, the composition comprises, as ingredient a), one or more synthetic dihydroxyflavanol derivative(s) that do not exist in nature.

According to another preferred embodiment of the invention, the composition of use in the process for dyeing keratin fibres comprises, as ingredient a), one or more natural dihydroxyflavanol derivative(s).

More particularly, the dihydroxyflavanol derivatives a) that may be used in the dyeing process according to the invention are in particular:

- dihydroxyflavanols such as catechin and epicatechin gallate, their oligomers and polymers called proanthocyanidols or condensed tannins such as theaflavin, theaflavin 3'-O-gallate, theaflavin 3,3'-O-digallate, and proanthocyanidins A1, A2, B1, B2, B3 and C1;

- and mixtures of the preceding compounds.

More preferably still, the dihydroxyflavanol derivatives are catechin and proanthocyanidins A1, A2, B1, B2, B3 and C1.
When the dyeing precursors have D and L forms, the two forms may be used in the compositions according to the invention, as may the racemic mixtures.
According to one embodiment, the natural dihydroxyflavanol derivatives are derived from extracts of animals, of bacteria, of fungi, of algae, of plants and of fruits, used in their entirety or partially. In particular regarding the plants, the extracts are derived from fruits, including citrus fruits, from vegetables, from trees and from shrubs. Mixtures of these extracts that are rich in dihydroxyflavanol derivatives as defined previously may also be used.
Preferably, the natural dihydroxyflavanol derivative(s) of the invention are derived from extracts of plants or plant parts.
For the purposes of the invention, these extracts will be put into the same category as compounds a).
The extracts are obtained by extraction from various plant parts, for instance the root, the wood, the bark, the leaf, the flower, the fruit, the seed, the pod or the peel.
Among the plant extracts, mention may be made of extracts of tea leaves and of rose.
Among the fruit extracts, mention may be made of extracts of apple, of grape (in particular of grape seed) or extracts of cocoa beans and/or pods.
Among the vegetable extracts, mention may be made of extracts of potato or of onion peel.
Among the extracts of tree wood, mention may be made of extracts of pine bark or extracts of logwood.
Mixtures of plant extracts may also be used.
According to a particular embodiment of the invention, the dihydroxyflavanol derivative(s) are natural extracts, rich in dihydroxyflavanol derivatives. According to a preferred form, the dihydroxyflavanol derivative(s) are solely natural extracts.
The natural extracts according to the invention may be in the form of powders or liquids. Preferably, the extracts of the invention are in the form of powders.
According to the invention, the natural, synthetic dihydroxyflavanol derivative(s) and/or the natural extract(s) used as ingredient a) in one or more composition(s) of use in the process according to the invention preferably represent(s) from 0.001% to 20% by weight of the total weight of the composition(s) containing the dihydroxyflavanol derivative(s) or the extract(s).
As regards the pure dihydroxyflavanol derivatives, the content in the composition(s) containing them is preferably between 0.001% and 5% by weight of each of these compositions.
As regards the extracts, the content in the composition(s) containing the extracts per se is preferably between 0.5% and 20% by weight of each of these compositions.
Preferably, the dihydroxyflavanol derivative is catechin.

Additional ortho-diphenol derivative

According to a particular embodiment of the invention, the dyeing process may use one or more ortho-diphenol derivative(s) other than the dihydroxyflavanol derivative(s) a).
The ortho-diphenol derivative(s) may be present in one or more cosmetic compositions used during the dyeing process.
A particular embodiment of the invention relates to ortho-diphenol derivatives or mixtures of compounds comprising one or more aromatic rings, preferably a benzene ring, comprising at least two hydroxyl (OH) groups carried by two adjacent carbon atoms of the aromatic ring.
The aromatic ring may more particularly be a fused aryl or fused heteroaromatic ring, i.e. optionally containing one or more heteroatoms, such as benzene, naphthalene, tetrahydronaphthalene, indane, indene, anthracene, phenanthrene, isoindole, indoline, isoindoline, benzofuran, dihydrobenzofuran, chroman, isochroman, chromene, isochromene, quinoline, tetrahydroquinoline and isoquinoline, said aromatic ring comprising at least two hydroxyl groups carried by two adjacent carbon atoms of the aromatic ring. Preferentially, the aromatic ring of the ortho-diphenol derivatives according to the invention is a benzene ring.
The term "fused ring" means that at least two saturated or unsaturated and heterocyclic or non-heterocyclic rings have a common bond, i.e. that at least one ring is placed beside another ring.
The ortho-diphenols according to the invention may or may not be salified. They may also be in aglycone form (without attached sugar) or in the form of glycosylated compounds.
More particularly, the ortho-diphenol derivative is different from the dihydroxyflavanol derivatives and is of formula (**II**), or an oligomer thereof, in salified or non-salified form:

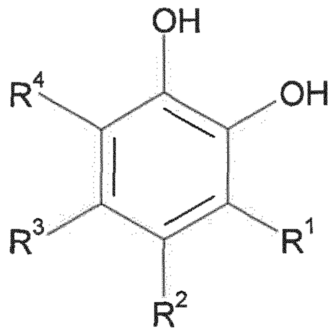

(II)

in which formula (**II**) the substituents:

**$R_1$** to **$R_4$**, which are identical or different, represent:

- a hydrogen atom,
- a halogen atom,
- a hydroxyl radical,
- a carboxyl radical,
- an alkoxycarbonyl radical,
- an optionally substituted amino radical,
- an optionally substituted linear or branched alkyl radical,
- an optionally substituted linear or branched alkenyl radical,
- an optionally substituted cycloalkyl radical,
- an alkoxy radical,
- an alkoxyalkyl radical,
- an alkoxyaryl radical, the aryl group possibly being optionally substituted,
- an aryl radical,
- a substituted aryl radical,
- a saturated or unsaturated heterocyclic radical, optionally carrying a cationic or anionic charge, optionally substituted and/or optionally fused with an aromatic ring, preferably a benzene ring, said aromatic ring being optionally substituted, particularly by one or more hydroxyl or glycosyloxy groups,
- a radical containing one or more silicon atoms,
- or two of the substituents carried by two adjacent carbon atoms **$R_1$ - $R_2$**, **$R_2$ - $R_3$** or **$R_3$ - $R_4$** form, together with the carbon atoms which carry them, a saturated or unsaturated, aromatic or non-aromatic ring, optionally containing one or more heteroatoms and optionally fused with one or more saturated or unsaturated rings optionally containing one or more heteroatoms. Particularly, **$R_1$** to **$R^4$** together form from one to four rings.

A particular embodiment of the invention concerns ortho-diphenol derivatives of formula (**II**) in which two adjacent substituents **$R_1$ - $R_2$**, **$R_2$ - $R_3$** or **$R_3$** - $R_4$ cannot form, with the carbon atoms which carry them, a pyrrolyl radical. More particularly, **$R_2$** and **$R_3$** cannot form a pyrrolyl radical fused to the benzene ring carrying the two hydroxyls.

The ortho-diphenols of use in the process of the invention may be natural or synthetic. Among the natural ortho-diphenols are compounds that may be present in nature and that are reproduced by chemical (semi)synthesis.

The ortho-diphenol salts of the invention may be salts of acids or bases. The acids may be inorganic or organic. Preferably, the acid is hydrochloric acid, which results in chlorides.

The bases may be inorganic or organic. In particular, the bases are alkali metal hydroxides, such as sodium hydroxide, which results in sodium salts.

According to a particular embodiment of the invention, the composition comprises one or more synthetic ortho-diphenol derivative(s) that do not exist in nature.

According to another preferred embodiment of the invention, the composition of use in the process for dyeing keratin fibres comprises one or more natural ortho-diphenol derivative(s).

More particularly, the ortho-diphenols that may be used in the process of the invention are in particular:

- anthocyanidins, for instance cyanidin, delphinidin or petunidin,
- anthocyanins or anthocyans, for instance myrtillin,
- ortho-hydroxybenzoates, for example gallic acid salts,

- hydroxystilbenes, for example 3,3',4,5'-tetrahydroxystilbene, optionally oxylated (for example glucosylated),
- 3,4-dihydroxyphenylalanine and derivatives thereof,
- 2,3-dihydroxyphenylalanine and derivatives thereof,
- 4,5-dihydroxyphenylalanine and derivatives thereof,
- dihydroxycinnamates, such as caffeic acid and chlorogenic acid,
- ortho-polyhydroxycoumarins,
- ortho-polyhydroxyisocoumarins,
- ortho-polyhydroxycoumarones,
- ortho-polyhydroxyisocoumarones,
- ortho-polyhydroxychalcones,
- ortho-polyhydroxychromones,
- quinones,
- hydroxyxanthones,
- 1,2-dihydroxybenzene and derivatives thereof,
- 1,2,4-trihydroxybenzene and derivatives thereof,
- 1,2,3-trihydroxybenzene and derivatives thereof,
- 2,4,5-trihydroxytoluene and derivatives thereof,
- proanthocyanidins and especially the proanthocyanidins A1, A2, B1, B2, B3 and C1,
- proanthocyanins,
- tannic acid,
- ellagic acid,
- and mixtures of the preceding compounds.

When the dyeing precursors have D and L forms, the two forms may be used in the compositions according to the invention, as may the racemic mixtures.

According to an embodiment, the natural ortho-diphenols are derived from extracts of animals, of bacteria, of fungi, of algae, of plants and of fruits, used in their entirety or partially. In particular regarding the plants, the extracts are derived from fruits, including citrus fruits, from vegetables, from trees and from shrubs. Mixtures of these extracts that are rich in ortho-diphenols as defined previously may also be used.

Preferably, the natural ortho-diphenol(s) of the invention are derived from extracts of plants or plant parts.

For the purposes of the invention, these extracts will be put into the same category as additional ortho-diphenol derivatives.

The extracts are obtained by extraction from various plant parts, for instance the root, the wood, the bark, the leaf, the flower, the fruit, the seed, the pod or the peel.

Among the plant extracts, mention may be made of extracts of tea leaves and of rose.

Among the fruit extracts, mention may be made of extracts of apple, of grape (in particular of grape seed) or extracts of cocoa beans and/or pods.

Among the vegetable extracts, mention may be made of extracts of potato or of onion peel.

Among the extracts of tree wood, mention may be made of extracts of pine bark or extracts of logwood.

Mixtures of plant extracts may also be used.

According to a particular embodiment of the invention, the ortho-diphenol derivative(s) are natural extracts, rich in ortho-diphenols. According to a preferred embodiment, the ortho-diphenol derivative(s) are solely natural extracts.

Preferentially, the ortho-diphenol(s) according to the invention are chosen from catechin, quercetin, haematein, haematoxylin, brasilin, gallic acid, and natural extracts containing them chosen from grape marc, pine bark, green tea, onion, cocoa bean, logwood, redwood and oak gall.

More preferably still, the ortho-diphenol(s) used in one or more compositions according to the invention are chosen from quercetin, gallic acid and haematoxylin.

The natural extracts according to the invention may be in the form of powders or liquids. Preferably, the extracts of the invention are in the form of powders.

According to the invention, the natural and synthetic ortho-diphenol derivative(s) and/or the natural extract(s) used as ingredient a) in one or more cosmetic composition(s) of use in the process according to the invention preferably represent(s) from 0.001% to 20% by weight of the total weight of the composition(s) containing the ortho-diphenol(s) or the extract(s).

As regards the pure ortho-diphenols, the content in the composition(s) containing them is preferably between 0.001% and 5% by weight of each of these compositions.

As regards the extracts, the content in the composition(s) containing the extracts per se is preferably between 0.5% and 20% by weight of each of these compositions.

b) manganese or zinc salt

The dyeing process according to the invention uses one or more manganese (Mn) salts or one or more zinc (Zn) salts.

For the purposes of the present invention, the term "manganese or zinc salts" means the oxides of these metals, such as manganese oxides, and the salts per se derived in particular from the action of an acid on a metal.

Preferably, the salts are not oxides.

Among the salts, mention may be made of halides, such as chlorides, fluorides and iodides; sulfates, phosphates; nitrates; perchlorates and carboxylic acid salts and polymer salts, and also mixtures thereof.

More particularly, the manganese salt is other than manganese carbonate, manganese hydrogencarbonate or manganese dihydrogencarbonate.

The carboxylic acid salts that may be used in the invention also include salts of hydroxylated carboxylic acids such as gluconate.

As examples of polymer salts, mention may be made of manganese pyrrolidonecarboxylate.

By way of example, mention may be made of manganese chloride, manganese fluoride, manganese acetate tetrahydrate, manganese lactate trihydrate, manganese phosphate, manganese iodide, manganese nitrate trihydrate, manganese bromide, manganese perchlorate tetrahydrate, manganese sulfate monohydrate and manganese gluconate. The salts advantageously used are manganese gluconate and manganese chloride.

Among the zinc salts, mention may be made of zinc sulfate, zinc gluconate, zinc chloride, zinc lactate, zinc acetate, zinc glycinate and zinc aspartate.

The manganese and zinc salts may be introduced in solid form into the compositions or may be derived from a natural, mineral or thermal water that is rich in these ions or alternatively from seawater (especially the Dead Sea). They may also originate from mineral compounds, for instance earths, ochres such as clays (for example green clay), or even from a plant extract containing them (cf., for example, patent document FR 2 814 943).

According to a preferred embodiment of the invention, the manganese or zinc salt(s) used represent(s) from 0.001% to 0.1% by weight, approximately, of the total weight of the composition(s) containing said metal salt(s), and even more preferentially from 0.05% to 10% by weight approximately.

In particular, the manganese and zinc salts employed in the invention are in oxidation state II, such as Mn(**II**) and Zn(**II**).

Preferably, the dyeing process of the invention employs one or more manganese salts, in particular Mn(**II**) metal salts.

More preferentially still, the manganese salt(s) is (are) chosen from carboxylic acid salts of manganese, in particular manganese gluconate; more particularly manganese gluconate.

c) hydrogen peroxide or hydrogen peroxide-generating system

In the context of the present invention, the third constituent is hydrogen peroxide or a hydrogen peroxide-generating system, such as:

a) urea hydrogen peroxide;
b) polymeric complexes that can release hydrogen peroxide, such as polyvinylpyrrolidone/$H_2O_2$, in particular in the form of powders, and the other polymeric complexes described in US 5 008 093, US 3 376 110 and US 5 183 901;
c) oxidases that produce hydrogen peroxide in the presence of a suitable substrate (for example glucose in the case of glucose oxidase or uric acid with uricase);
d) metal peroxides that generate hydrogen peroxide in water, for instance calcium peroxide or magnesium peroxide;
e) perborates; or
f) percarbonates.

According to a preferred embodiment of the invention, the composition contains one or more hydrogen peroxide-generating system(s) chosen from a) urea hydrogen peroxide, b) polymeric complexes that can release hydrogen peroxide chosen from polyvinylpyrrolidone/$H_2O_2$, c) oxidases, e) perborates and f) percarbonates.

In particular, the third constituent is hydrogen peroxide.

Moreover, the composition(s) comprising hydrogen peroxide or the hydrogen peroxide generator may also contain various adjuvants conventionally used in hair dyeing compositions and as defined hereinbelow.

According to a particular form of the invention, the hydrogen peroxide used or the hydrogen peroxide-generating system(s) used preferably represent(s) from 0.001% to 12% by weight of hydrogen peroxide relative to the total weight of the composition(s) containing it/them, and even more preferentially from 0.2% to 2.7% by weight.

d) (bi)carbonate(s) or (bi)carbonate-generating system

In accordance with the present invention, the dyeing process uses one or more (bi)carbonates or one or more (bi)carbonate-generating systems.

The term "(bi)carbonate-generating system" means a system which generates (bi)carbonate *in situ,* for instance carbon dioxide in water or by buffering carbonate with an inorganic or organic acid. The (bi)carbonate(s) or the (bi)carbonate-generating system(s) may be used in one or more cosmetic compositions during the dyeing process.

The (bi)carbonate(s) is (are) chosen from:

a) carbonates of alkali metals ($Met^{+}{}_2CO_3{}^{2-}$), of alkaline earth metals ($Met'^{2+}CO_3{}^{2-}$), of ammonium ($(R''_4N')_2CO_3{}^{2-}$) or of phosphonium ($(R''_4P^+)_2CO_3{}^{2-}$) with Met' representing an alkaline earth metal and Met representing an alkali metal, and R", which are identical or different, representing a hydrogen atom or an optionally substituted ($C_1$-$C_6$)alkyl group, such as hydroxyethyl,

b) bicarbonates, also known as hydrogencarbonates, of the following formulae:

- $R'^{+}HCO_3{}^{-}$ with R' representing a hydrogen atom, an alkali metal, an ammonium group $R''_4N^+$- or a phosphonium group $R''_4P^+$- in which R", which are identical or different, represent a hydrogen atom or an optionally substituted ($C_1$-$C_6$)alkyl group, such as hydroxyethyl, and, when R' represents a hydrogen atom, the hydrogencarbonate is then known as dihydrogencarbonate ($CO_2$, $H_2O$); and
- $Met'^{2+}$ $(HCO_3{}^{-})_2$ with Met' representing an alkaline earth metal.

More particularly, the (bi)carbonate(s) is (are) chosen from alkali metal, alkaline earth metal or ammonium (bi)carbonates; preferentially, alkali metal or ammonium (bi)carbonates.

Mention may be made of Na, K, Mg and Ca carbonates or hydrogencarbonates and mixtures thereof, and in particular of sodium hydrogencarbonate. These hydrogencarbonates may originate from a natural water, for example spring water from the Vichy basin or from La Roche-Posay or Badoit water (cf., for example, patent document FR 2 814 943). In particular, mention may be made of sodium carbonate [497-19-8] = $Na_2CO_3$, sodium hydrogencarbonate or sodium bicarbonate [144-55-8] = $NaHCO_3$, and sodium dihydrogencarbonate = $Na(HCO_3)_2$.

According to the invention, the (bi)carbonate agent(s) used preferably represent(s) from 0.001% to 10% by weight of the total weight of the composition(s) containing the (bi)carbonate agent(s) and even more preferentially from 0.005% to 5% by weight.

Preferably, the ingredient d) is a (bi)carbonate. More preferably, the (bi)carbonate used in the composition during the dyeing process is ammonium (bi)carbonate or sodium bicarbonate.

e) alkalinizing agent(s) other than the bicarbonate(s)

The alkalinizing agent used in the dyeing process according to the invention as fifth ingredient is different from the (bi)carbonate(s) d) as defined previously. It is an agent which makes it possible to increase the pH of the composition(s) in which it is present. The alkalinizing agent is a Bronsted, Lowry or Lewis base. It may be inorganic or organic.

In particular, said agent is chosen from i) aqueous ammonia, ii) alkanolamines such as monoethanolamine, diethanolamine, triethanolamine and derivatives thereof, iii) oxyethylenated and/or oxypropylenated ethylenediamines, iv) inorganic or organic hydroxides, v) alkali metal silicates such as sodium metasilicates, vi) amino acids, preferably basic amino acids such as arginine, lysine, ornithine, citrulline and histidine, and vii) the compounds of formula **(III)** below:

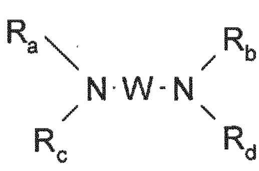

**(III)**

in which:

- W is a divalent ($C_1$-$C_8$)alkylene group, preferably a propylene group, optionally substituted in particular by a hydroxyl group or a Ci-$C_4$ alkyl radical;
- $R_a$, $R_b$, $R_c$ and Rd, which are identical or different, represent a hydrogen atom or a $C_1$-$C_4$ alkyl or $C_1$-$C_4$ hydroxyalkyl radical.

The inorganic or organic hydroxides are preferably chosen from i) hydroxides of an alkali metal, ii) hydroxides of an alkaline earth metal, for instance sodium hydroxide or potassium hydroxide, iii) hydroxides of a transition metal, such as hydroxides of metals from Groups III, IV, V and VI, and iv) hydroxides of lanthanides or of actinides, quaternary ammonium hydroxides and guanidinium hydroxide.

Preferentially, whether for the composition according to the invention or the process implemented in the invention, the alkalinizing agent is not sodium hydroxide NaOH. More generally and preferentially, whether for the composition according to the invention or the process implemented in the invention, the alkalinizing agent is not an alkali metal hydroxide XOH, with X = alkali metal.

The hydroxide may be formed *in situ,* for instance guanidine hydroxide, by reacting calcium hydroxide and guanidine carbonate.

The alkalinizing agent(s) e) as defined previously preferably represent(s) from 0.001% to 10% by weight of the weight of the composition(s) containing them, more particularly from 0.005% to 8% by weight of the composition.

Preferably, the alkaline agent(s) is (are) chosen from alkanolamines, in particular monoethanolamine.

f) <u>magnesium, molybdenum or calcium salts</u>

In accordance with the present invention, the dyeing process according to the invention additionally employs one or more metal salt(s) chosen from magnesium salts, molybdenum salts and calcium salts.

The term "magnesium, molybdenum or calcium salt" is understood to mean, within the meaning of the present invention, the oxides of these metals and the salts properly speaking resulting in particular from an acid with a metal.

Preferably, the salts of these metals are not oxides.

Among the salts of these metals, mention may be made of hydroxides, halides, sulfates, phosphates, nitrates and carboxylic acid salts and polymer salts, and also mixtures thereof.

The carboxylic acid salts that may be used in the invention also include salts of hydroxylated carboxylic acids such as gluconate.

According to a particular embodiment of the invention, the metal derivatives(s) is (are) magnesium (Mg) derivative(s).

Mention may be made, among magnesium salts, of hydroxides, such as magnesium hydroxide, halides, sulfates, nitrates, such as magnesium nitrate, or carboxylic acid salts.

In particular, the carboxylic acid salts of magnesium are chosen from magnesium acetate, magnesium oxalate, magnesium citrate, magnesium lactate, magnesium gluconate, magnesium ascorbate, magnesium formate dihydrate or magnesium stearate.

In particular, the halides are chosen from magnesium chloride, which may be hydrated or anhydrous, magnesium bromide or magnesium iodide.

In particular, the sulfates are hydrated or anhydrous magnesium sulfate.

In particular, the phosphates are magnesium phosphate, magnesium hydrogenphosphate or tribasic magnesium phosphate $Mg_3(PO_4)_2$, which may be hydrated or anhydrous.

In particular, the magnesium salt is Dead Sea Salt [7647-14-5] obtained by evaporation of water from the Dead Sea, such as sold by Tri Industries under the name "Dead Sea Salt" or by Sea Works Ltd under the name "Dead Sea Bath Salt".

Preferably, the magnesium salts are chosen from magnesium sulfates, carboxylic acid salts, in particular magnesium gluconate and magnesium acetate, and Dead Sea salt.

In a preferred form of the invention, the amount in moles of magnesium salts used in the process or in the composition is equivalent in moles to that of the dyes.

The magnesium salt(s) is (are) present in the cosmetic composition(s) used in the process according to the invention in a content ranging from 0.001% to 20% by weight, relative to the total weight of the composition(s) in which they are present.

According to a particular embodiment of the invention, the metal derivative(s) is (are) molybdenum (Mo) derivative(s).

In particular, the molybdenum metal derivative(s) exhibit an oxidation state ranging from (II) to (VI) and are as described in Kirk-Othmer's Encyclopedia of Chemical Technology Copyright © 2001 by John Wiley & Sons Inc. Last updated: 17 Sep 2009, "Molybdenum compounds", Edward I. Stieffel, pp. 871-895, or Ullmann's Encyclopedia; WILEY-VCH Verlag GmbH & Co. KGaA, 2000-2005, "Molybdenum and Molybdenum Compounds".

More particularly:

a) binary molybdenum halides of oxidation states (II) to (VI), hexacoordinated with 6 halogens of Mo(V), (IV) and (III), the molybdenum atoms being bonded via halogen bonds such as Mo(II) halide, containing $[Mo_6Hal_8]^{4+}$ clusters bonded to halogen atoms to give $Mo_6Hal_{12}$ with Hal, which are identical or different, representing a halogen atom,

and more particularly Hal represents Cl;
b) molybdenum tetrahalides $(Hal')_4Mo$ with Hal', which are identical or different, representing a halogen atom, in particular $MoCl_4$;
c) molybdenum sulfur derivatives chosen from:

- molybdenum disulfides [*1317-33-5*], molybdenum(IV) sulfides, $MoS_2$;
- molybdenum sesquisulfide [*12033-33-9*]; dimolybdenum(III) trisulfides, $Mo_2S_3$;

d) molybdenum acetate dimer [14221-06-8].

In a preferred form of the invention, the amount in moles of molybdenum salts used in the process or in the composition is equivalent in moles to that of the dyes.
Preferably, the molybdenum metal derivative is chosen from molybdenum acetate dimer.
The molybdenum salt(s) is (are) present in the cosmetic composition(s) used in the process according to the invention in a content ranging from 0.001% to 20% by weight, relative to the total weight of the composition(s) in which they are present.
According to a particular embodiment of the invention, the metal derivative(s) is (are) chosen from calcium salts and also the hydrates thereof and supported forms thereof.
By way of example, mention may be made of the hydroxides, sulfates, halides, carboxylates, phosphates, borates and carbonates.
In particular, the hydroxides are calcium hydroxide $Ca(OH)_2$.
In particular, the sulfates are chosen from calcium sulfate $CaSO_4$ or gypsum.
In particular, the halides are chosen from calcium chloride, calcium bromide or calcium iodide.
In particular, the carboxylates are chosen from calcium oxalate, calcium diacetate, calcium citrate, tricalcium 2-hydroxypropanoate-1,2,3-tricarboxylate [813-94-5], calcium lactate, calcium 2-hydroxypropanoate, hydrated or anhydrous calcium gluconate, calcium ascorbate, calcium 2-ethylbutanoate, calcium formate, calcium levulinate, calcium mesoxalate, calcium oleate, calcium palmitate, calcium propionate, calcium saccharate, calcium stearate, calcium stearoyl-2-lactylate, calcium succinate, calcium tartrate and calcium pyrrolidonecarboxylate.
In particular, the calcium phosphates are chosen from calcium phosphate $Ca_3(PO_4)_2$, hydrated or anhydrous calcium hydrogenphosphate $CaHPO_4$, calcium hydroxyphosphate $[Ca_5(OH)(PO_4)_3]$, hydrated or anhydrous calcium bis(dihydrogenphosphate) $Ca(H_2PO_4)_2$, calcium pyrophosphate $Ca_2P_2O_7$, hydroxyapatite, calcium fluorophosphate and calcium glycerophosphate.
In particular, the calcium derivative is calcium borate.
Preferentially, the calcium derivative is chosen from calcium sulfate $CaSO_4$, gypsum, calcium diacetate, hydrated or anhydrous calcium gluconate, calcium phosphate $Ca_3(PO_4)_2$, hydrated or anhydrous calcium hydrogenphosphate $CaHPO_4$, hydrated or anhydrous calcium bis(dihydrogenphosphate) $Ca(H_2PO_4)_2$, calcium pyrrolidonecarboxylate and hydroxyapatite.
More preferably still, the calcium salts are chosen from calcium carboxylates.
In particular, the calcium salts are chosen from calcium acetate monohydrate, calcium gluconate hydrate and calcium pyrrolidonecarboxylate.
In a preferred form of the invention, the amount in moles of calcium salts used in the process or in the composition is equivalent in moles to that of the dyes.
The calcium salt(s) is (are) present in the cosmetic composition(s) used in the process according to the invention in a content ranging from 0.001% to 20% by weight, relative to the total weight of the composition(s) in which they are present.
Preferentially, the metal salts f) according to the invention are soluble in water to a proportion of at least 0.0001 g/l.
The metal salts f) according to the invention may be introduced in solid form into the compositions or may originate from a natural, mineral or thermal water that is rich in these ions, or alternatively from seawater (in particular Dead Sea water). They may also originate from inorganic compounds, such as plant extracts containing them (cf., for example, patent document FR 2 814 943).

- water:

Water is included in the process of the invention. It originates from the composition comprising the ingredient a).

- cosmetic compositions:

The cosmetic compositions according to the invention are cosmetically acceptable, i.e. they comprise a dyeing

support that contains water or a mixture of water and of one or more organic solvents or a mixture of organic solvents.

The term "organic solvent" means an organic substance that is capable of dissolving or dispersing another substance without chemically modifying it.

- organic solvents:

Examples of organic solvents that may be mentioned include lower $C_1$-$C_4$ alkanols, such as ethanol and isopropanol; polyols and polyol ethers such as 2-butoxyethanol, propylene glycol, propylene glycol monomethyl ether, diethylene glycol monoethyl ether, diethylene glycol monomethyl ether or hexylene glycol; and also aromatic alcohols, for instance benzyl alcohol or phenoxyethanol.

The organic solvents are present in proportions preferably of between 1% and 40% by weight approximately and more preferably still between 5% and 30% by weight approximately relative to the total weight of the dyeing composition.

- adjuvants:

The composition(s) of the dyeing process in accordance with the invention may also contain various adjuvants conventionally used in hair dyeing compositions, such as anionic, cationic, non-ionic, amphoteric or zwitterionic surfactants or mixtures thereof, anionic, cationic, non-ionic, amphoteric or zwitterionic polymers or mixtures thereof, inorganic or organic thickeners, and in particular anionic, cationic, non-ionic and amphoteric polymeric associative thickeners, antioxidants, penetrating agents, sequestering agents, fragrances, buffers, dispersing agents, conditioning agents, for instance volatile or non-volatile and modified or unmodified silicones, film-forming agents, ceramides, preservatives and opacifying agents.

Said adjuvants are preferably chosen from surfactants such as anionic or non-ionic surfactants or mixtures thereof and inorganic or organic thickeners.

The above adjuvants are generally present in an amount for each of them of between 0.01% and 40% by weight relative to the weight of the composition, and preferably between 0.1% and 20% by weight relative to the weight of the composition.

Needless to say, those skilled in the art will take care to select this or these optional additional compound(s) such that the advantageous properties intrinsically associated with the composition(s) of use in the dyeing process in accordance with the invention are not, or are not substantially, adversely affected by the envisioned addition(s).

- additional dyes:

The dyeing process using the ingredients a) to f) as previously defined may also use one or more additional direct dyes. These direct dyes are chosen, for example, from those conventionally used in direct dyeing, and among which mention may be made of any commonly used aromatic and/or non-aromatic dye such as neutral, acidic or cationic nitrobenzene direct dyes, neutral, acidic or cationic azo direct dyes, natural direct dyes other than ortho-diphenols, neutral, acidic or cationic quinone and in particular anthraquinone direct dyes, azine, triarylmethane, indoamine, methine, styryl, porphyrin, metalloporphyrin, phthalocyanine and methine cyanine direct dyes, and fluorescent dyes.

Among the natural direct dyes, mention may be made of lawsone, juglone, indigo, isatin, curcumin, spinulosin, apigenidin and orceins. Extracts or decoctions containing these natural dyes and in particular cataplasms or henna-based extracts may also be used.

According to the invention, the direct dye(s) used in the composition according to the invention which comprises the ingredients a) to f) as previously defined, or the composition(s) of the dyeing process according to the invention, preferably represents from 0.001% to 10% by weight approximately of the total weight of the composition(s) and even more preferentially from 0.05% to 5% by weight approximately.

The composition according to the invention or the composition(s) of the process using the ingredients a) to f) as previously defined may also comprise one or more oxidation bases and/or one or more couplers conventionally used for dyeing keratin fibres.

Among the oxidation bases, mention may be made of para-phenylenediamines, bis(phenyl)alkylenediamines, para-aminophenols, bis-para-aminophenols, ortho-aminophenols and heterocyclic bases, and the addition salts thereof.

Among these couplers, mention may be made especially of meta-phenylenediamines, meta-aminophenols, meta-diphenols, naphthalene couplers and heterocyclic couplers, and the addition salts thereof.

The oxidation base(s) present in said composition(s) that is (are) used in the process is (are) generally each present in an amount of between 0.001% and 10% by weight of the total weight of the composition(s) containing it or them.

The cosmetic composition(s) of the invention may be in various formulation forms, such as a powder, a lotion, a mousse, a cream or a gel, or in any other form that is suitable for dyeing keratin fibres. They may also be packaged

in a propellant-free pump-action spray or under pressure in an aerosol container in the presence of a propellant and form a foam.

- pH of the composition(s):

In accordance with the present invention, at least one of the compositions used during the dyeing process of the present invention has an alkaline pH, i.e. greater than 7, and preferably between 8 and 12, and more particularly between 8 and 10.

According to a particular embodiment of the invention, the pH of the composition comprising d) one or more (bi)carbonate(s) or one or more (bi)carbonate-generating system(s) and e) one or more alkalinizing agent(s) other than the (bi)carbonate(s) is alkaline, i.e. greater than 7, preferably between 8 and 12, and more particularly between 8 and 10.

In other words, the (bi)carbonate(s) or the (bi)carbonate-generating system(s) d) and the alkalinizing agent(s) other than the (bi)carbonate(s) e) is (are) used in a single composition and the pH of said composition is preferably alkaline, i.e. greater than 7, preferably between 8 and 12, and more particularly between 8 and 10, in particular between 9 and 10.

Even more preferentially, the (bi)carbonate(s) chosen from ammonium bicarbonates and the alkalinizing agents chosen from alkanolamines, in particular monoethanolamine, are used in a single composition and the pH of said composition is alkaline, i.e. greater than 7, preferably between 8 and 12, and more particularly between 8 and 10, in particular between 9 and 10.

Preferably, the composition containing the dihydroxyflavanol derivative(s) and not containing (bi)carbonates has (have) a pH of less than 7 and preferably of between 3 and 6.5.

The pH of this compositions may be adjusted to the desired value by means of alkalinizing agents as defined previously in d) or using acidifying agents usually used in the dyeing of keratin fibres, or alternatively by means of standard buffer systems.

Among the acidifying agents for the compositions used in the invention, examples that may be mentioned include inorganic or organic acids, for instance hydrochloric acid, orthophosphoric acid, sulfuric acid, carboxylic acids, for instance acetic acid, tartaric acid, citric acid or lactic acid, or sulfonic acids.

Among the alkalinizing agents concerned are the agents as defined previously under the heading "e) alkalinizing agent(s)".

- dyeing process in two steps

The process of the present invention is a two-step process, the first step consists in applying to the keratin fibres a dyeing composition comprising the ingredients a), b), c) and f) as defined previously, and then, in a second step, a composition comprising the ingredients d) and e) as defined previously is applied to said keratin fibres, it being understood that at least one of the two compositions is aqueous. The composition comprising the dihydroxyflavanol derivative(s) is aqueous. Even more preferentially, the two compositions used in this embodiment are aqueous.

The leave-on time after application for the first step is generally set at between 3 and 120 minutes, preferentially between 10 and 60 minutes and more preferentially between 15 and 45 minutes. The application time of the composition used during the second step is generally set at between 3 and 120 minutes, preferentially between 3 and 60 minutes and more preferentially between 5 and 30 minutes.

The metal salts f) are used in co-treatment of the keratin fibres with the manganese or zinc salts.

More particularly, the dihydroxyflavanol derivative employed in the composition of the first step is catechin.

The application temperature is generally between ambient temperature (15 to 25°C) and 80°C and more particularly between 15 and 45°C. Thus, after application of the composition according to the invention, the head of hair may advantageously be subjected to a heat treatment by heating to a temperature of between 30 and 60°C. In practice, this operation may be performed using a styling hood, a hairdryer, an infrared ray dispenser or other standard heating appliances.

Use may be made, both as means for heating and smoothing the hair, of a heating iron at a temperature of between 60°C and 220°C and preferably between 120°C and 200°C.

The keratin fibres may optionally be mechanically wiped and/or dried between each step, in particular before carrying out the step comprising the application of the composition comprising the ingredient d).

The steps of intermediate mechanical wiping and drying are also known as "controlled leave-in" to distinguish from "standard copious rinsing with water" and "leave-in". The term "mechanical wiping" of the fibres means rubbing an absorbent article on the fibres and the physical removal, by means of the absorbent article, of the excess ingredient(s) that have not penetrated into the fibres. The absorbent article may be a piece of fabric such as a towel, particularly a terry towel, a cloth or absorbent paper such as household roll towel.

According to a particularly advantageous process of the invention, the mechanical wiping is performed without

total drying of the fibre, leaving the fibre moist.

The term "drying" means the action of evaporating the organic solvents and/or water that are in one or more compositions used in the process of the invention, comprising or not comprising one or more ingredients a) to f) as defined previously. The drying may be performed with a source of heat (convection, conduction or radiation) by sending, for example, a stream of hot gas such as air necessary to evaporate the solvent(s). Sources of heat that may be mentioned include a hairdryer, a hairstyling hood, a hair-smoothing iron, an infrared ray dispenser or other standard heating appliances.

A particular form of the invention concerns a dyeing process that is performed at ambient temperature (25°C). The compositions mentioned are ready-for-use compositions.

The present invention also relates to the use of one or more metal salt(s) f) as defined previously for improving the chromaticity of keratin fibres, in particular human keratin fibres such as the hair, coloured using one or more dihydroxyflavanol derivative(s) as defined previously.

In particular, the invention relates to the use of one or more metal salt(s) f) in a dyeing composition comprising one or more dihydroxyflavanol derivative(s) for improving the chromaticity of keratin fibres.

The following examples serve to illustrate the invention without, however, being limiting in nature.

I. Dyeing example

In the following example, the chromaticity C* was compared between a dyeing process according to the invention and a dyeing process which is identical but which does not involve the use of magnesium salts.

1. Compositions tested

| **Compositions** | **A1** | **A2** | **A3** | **A4** |
|---|---|---|---|---|
| Catechin a) | 0.420 g | 0.420 g | 0.420 g | 0.420 g |
| Manganese gluconate b) | 0.003 g | 0.003 g | 0.003 g | 0.003 g |
| Magnesium sulfate f) | 0.18 g | - | - | - |
| Magnesium acetate tetrahydrate f) | - | 0.32 g | - | - |
| Dead Sea salt f) | - | - | 1.492 mmol** | - |
| Magnesium gluconate f) | - | - | - | 0.29 g |
| Hydrogen peroxide* c) | 1.2 g | 1.2 g | 1.2 g | 1.2 g |
| Ethanol | 13.2 g | 13.2 g | 13.2 g | 13.2 g |
| Propylene glycol | 13.2 g | 13.2 g | 13.2 g | 13.2 g |
| Water | q.s. for 100 g | q.s. for 100 g | q.s. for 100 g | q.s. for 100 g |
| (*) 0.144 g of 167-volume aqueous hydrogen peroxide solution<br>(**) Dead Sea salt comprising 33% by weight of magnesium chloride | | | | |

| **Composition B** (developer) | Amount |
|---|---|
| Sodium bicarbonate d) | 2.6 g |
| Monoethanolamine e) | 2 g |
| Demineralized water | q.s. for 100 g |

2. Procedure

Each composition **A1, A2, A3** and **A4** is applied to locks of hair comprising 90% white hairs that have been permanent-waved, in a proportion of 12 grams of composition per gram of hair. The composition is then left to stand on the locks for 30 minutes at a temperature of 50°C.

After this, the impregnated hair is wiped using an absorbent paper towel to remove the excess composition.

The composition **B** is then applied to each of the locks in a proportion of 4 grams of composition per gram of lock. The composition B exhibits a pH = 9.5. The leave-on time is 10 minutes at ambient temperature. The hair is then rinsed with water, washed with a conventional shampoo and dried under a hood.

3. Colorimetric results

The chromaticity C* is compared between a dyeing process according to the invention and an identical process not employing the magnesium salt.

The colour of the locks was evaluated in the CIE L* a* b* system, using a Minolta Spectrophotometer CM2600D colorimeter. In this L* a* b* system, the three parameters denote, respectively, the colour intensity (L*), the green/red colour axis (a*) and the blue/yellow colour axis (b*).

The chromaticity was measured by means of the values a* and b*, and is obtained from the following formula:

$$C^* = \sqrt{a^{*2} + b^{*2}} \qquad \text{(III)}$$

The higher the C* value, the more chromatic is the colour obtained.

The values of the chromaticity C* are given in the table below:

| Results | C* |
|---|---|
| Without magnesium salts (comparative) | 19.8 |
| Magnesium sulfate (invention) | 21.5 |
| Magnesium acetate tetrahydrate (invention) | 21.0 |
| Magnesium gluconate (invention) | 23.2 |
| Dead Sea salt (invention) | 21.3 |

It is found that the dyeing process according to the invention makes it possible to result in more chromatic colourations than the comparative process devoid of metal salt f).

II. Dyeing example

In the following example, the chromaticity C* was compared between a dyeing process according to the invention and a dyeing process which is identical but which does not involve the use of a molybdenum salt.

1. Compositions tested

| Composition | A5 |
|---|---|
| Catechin a) | 0.420 g |
| Manganese gluconate b) | 0.003 g |
| Molybdenum acetate dimer f) | 0.64 g |
| Hydrogen peroxide c) | 1.2 g |
| Ethanol | 13.2 g |
| Propylene glycol | 13.2 g |
| Water | q.s. for 100 g |

| **Composition B** (developer) | Amount |
|---|---|
| Ammonium bicarbonate d) | 2.6 g |
| Monoethanolamine e) | 2 g |
| Demineralized water | q.s. for 100 g |

2. Procedure

The composition **A5** is applied to locks of hair comprising 90% white hairs that have been permanent-waved, in a proportion of 12 grams of composition per gram of hair. The composition is then left to stand on the locks for 30 minutes at a temperature of 50°C.

After this, the impregnated hair is wiped using an absorbent paper towel to remove the excess composition.

The composition **B** is then applied to the locks in a proportion of 4 grams of composition per gram of lock. The composition **B** exhibits a pH = 9.5. The leave-on time is 10 minutes at ambient temperature. The hair is then rinsed with water, washed with a conventional shampoo and dried under a hood.

3. Colorimetric results

The chromaticity C* is calculated in accordance with equation (III) and its values are given in the table below:

| **Results** | C* |
|---|---|
| Without molybdenum salts (comparative) | 19.8 |
| Molybdenum acetate dimer (invention) | 21.5 |

It is found that the dyeing process according to the invention makes it possible to result in more chromatic colourations than the comparative process devoid of metal salt f).

III. Dyeing example

In the following example, the chromaticity C* was compared between a dyeing process according to the invention and a dyeing process which is identical but which does not involve the use of a composition comprising calcium salts.

1. Compositions tested

| **Compositions** | **A6** | **A7** | **A8** |
|---|---|---|---|
| Catechin a) | 0.420 g | 0.420 g | 0.420 g |
| Manganese gluconate b) | 0.003 g | 0.003 g | 0.003 g |
| Calcium acetate monohydrate f) | 0.26 g | - | - |
| Calcium pyrrolidonecarboxylate f) | - | 0.44 g | - |
| Calcium gluconate f) | | | 0.64 g |
| Hydrogen peroxide c) | 1.2 g | 1.2 g | 1.2 g |
| Ethanol | 13.2 g | 13.2 g | 13.2 g |
| Propylene glycol | 13.2 g | 13.2 g | 13.2 g |
| Water | q.s. for 100 g | q.s. for 100 g | q.s. for 100 g |

| Composition B | Developer |
|---|---|
| Ammonium bicarbonate d) | 2.6 g |
| Monoethanolamine e) | 2 g |
| Demineralized water | q.s. for 100 g |

2. Procedure

Each composition **A6, A7** and **A8** is applied to locks of hair comprising 90% white hairs that have been permanent-waved, in a proportion of 12 grams of composition per gram of hair. The composition is then left to stand on the locks for 30 minutes at a temperature of 50°C.

After this, the impregnated hair is wiped using an absorbent paper towel to remove the excess composition.

The composition **B** is then applied to each of the locks in a proportion of 4 grams of composition per gram of lock. The composition B exhibits a pH = 9.5. The leave-on time is 10 minutes at ambient temperature. The hair is then rinsed with water, washed with a conventional shampoo and dried under a hood.

3. Colorimetric results

The chromaticity C* is calculated in accordance with equation (III) and the values are given in the table below:

| Results | C* |
|---|---|
| Without calcium salts (comparative) | 19.8 |
| Calcium acetate monohydrate (invention) | 23.7 |
| Calcium pyrrolidonecarboxylate (invention) | 21.2 |
| Calcium gluconate (invention) | 21.8 |

It is found that the dyeing process according to the invention makes it possible to result in more chromatic colourations than the comparative process devoid of metal salt f).

## Claims

**1.** Process for dyeing keratin fibres, in particular human keratin fibres such as the hair, in which:

a first step consists in applying to said keratin fibres a composition comprising the following ingredients:

a) one or more dihydroxyflavanol derivative(s), represented by compounds of following formula (I) and also organic or inorganic acid salts thereof, optical, geometric and tautomeric isomers thereof, and solvates thereof such as the hydrates:

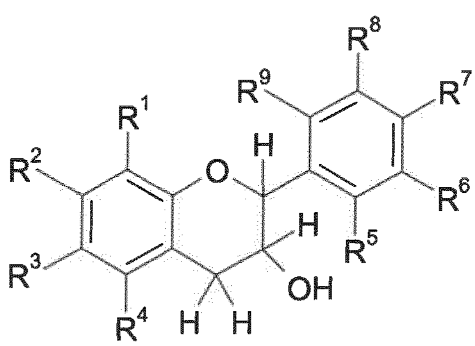

(I)

in which:

• $R^1$, $R^2$, $R^3$, $R^4$, $R^5$, $R^6$, $R^7$, $R^8$ and $R^9$, which are identical or different, represent:

- a hydrogen atom,
- a halogen atom,
- a hydroxyl group,
- a $(C_1-C_6)$alkyl group,
- a $(C_1-C_6)$alkoxy group,
- a $(C_1-C_6)$alkylthio group,
- a carboxyl group,
- an alkoxycarbonyl group,
- an optionally substituted amino group,
- an optionally substituted linear or branched alkenyl group,
- an optionally substituted cycloalkyl group,
- an aryl group,
- a substituted aryl group,
- a group containing one or more silicon atoms,
- a (di)((hydroxy)$(C_1-C_6)$alkyl)amino group,
- an -O-sugar group, such as -O-glucoside,
- an R-Z-C(X)-Y- group with R representing a hydrogen atom or a $(C_1-C_6)$alkyl group, Y and Z, which may be identical or different, representing a bond, an oxygen or sulfur atom or an -N(R')- group with R' representing a hydrogen atom or a $(C_1-C_6)$alkyl group, Y possibly also representing a $(C_1-C_6)$alkylene group, and X representing an oxygen or sulfur atom, or N-R" with R" representing a hydrogen atom or a $(C_1-C_6)$alkyl group;

it being understood that at least two adjacent radicals chosen from $R^1$, $R^2$, $R^3$, $R^4$, $R^5$, $R^6$, $R^7$, $R^8$ and $R^9$ represent a hydroxyl group,

b) one or more manganese salt(s) or one or more zinc salt(s),
c) hydrogen peroxide or one or more hydrogen peroxide-generating system(s), and
f) one or more metal salt(s) chosen from magnesium salts, molybdenum salts and calcium salts;

and then, a second step consists in applying to said keratin fibres a composition comprising the following ingredients:

d) one or more (bi)carbonate(s) or one or more (bi)carbonate-generating system(s), and
e) one or more alkalinizing agent(s) other than the bicarbonate(s);

it being understood that:

- at least one of the compositions used during said dyeing process has an alkaline pH, i.e. greater than 7, and
- the composition comprising the ingredient a) is aqueous.

**2.** Dyeing process according to Claim 1, **characterized in that** the dihydroxyflavanol derivative(s) is (are) chosen from natural dihydroxyflavanol derivative(s).

**3.** Dyeing process according to any one of Claims 1 or 2, **characterized in that** the dihydroxyflavanol derivative(s) is (are) chosen from:

- dihydroxyflavanols such as catechin and epicatechin gallate, their oligomers and polymers called proanthocyanidols or condensed tannins such as theaflavin, theaflavin 3'-O-gallate, theaflavin 3,3'-O-digallate, and proanthocyanidins A1, A2, B1, B2, B3 and C1;
- and mixtures of the preceding compounds.

**4.** Dyeing process according to any one of Claims 2 or 3, **characterized in that** the natural dihydroxyflavanol derivative(s) is (are) chosen from extracts of animals, of bacteria, of fungi, of algae, of plants and of fruits.

**5.** Dyeing process according to any one of the preceding claims, **characterized in that**:

- the manganese salts are chosen from:

i) manganese oxides and
ii) halides, sulfates, phosphates, nitrates and perchlorates, carboxylic acid salts, such as gluconates, and also mixtures thereof, and

- the zinc salts are chosen from zinc sulfate, zinc gluconate, zinc chloride, zinc lactate, zinc acetate, zinc glycinate and zinc aspartate;

and, more particularly, the salts b) are carboxylic acid salts of manganese.

**6.** Dyeing process according to any one of the preceding claims, **characterized in that** the ingredient c) is hydrogen peroxide or urea hydrogen peroxide.

**7.** Dyeing process according to any one of the preceding claims, **characterized in that** the (bi)carbonate(s) is (are) alkali metal, alkaline earth metal or ammonium (bi)carbonate(s).

**8.** Dyeing process according to any one of the preceding claims, **characterized in that** the alkaline agent(s) other than the bicarbonate(s) is (are) chosen from:

- aqueous ammonia,
- alkanolamines,
- oxyethylenated ethylenediamines,
- oxypropylenated ethylenediamines,
- inorganic or organic hydroxides,
- alkali metal silicates,
- amino acids, and
- the compounds of formula (**III**) below:

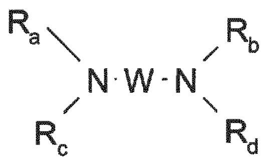

(III)

in which formula (**III**):

- W is a divalent ($C_1$-$C_8$)alkylene group, preferably a propylene group, optionally substituted in particular by a hydroxyl group or a $C_1$-$C_4$ alkyl radical;
- $R_a$, $R_b$, $R_c$ and $R_d$, which are identical or different, represent a hydrogen atom or a $C_1$-$C_4$ alkyl or $C_1$-$C_4$ hydroxyalkyl radical;

preferably, the alkaline agent(s) e) is (are) chosen from alkanolamines, in particular monoethanolamine.

**9.** Dyeing process according to any one of the preceding claims, **characterized in that**:

- the magnesium salts are chosen from magnesium hydroxides, magnesium halides, magnesium sulfates, magnesium nitrates, carboxylic acid salts of magnesium, and magnesium phosphates,
- the molybdenum salts are chosen from:

i) binary molybdenum halides of oxidation states (II) to (VI), hexacoordinated with 6 halogens of Mo(V), (IV) and (III), the molybdenum atoms being bonded via halogen bonds such as Mo(II) halide, containing $[Mo_6Hal_8]^{4+}$ clusters bonded to halogen atoms to give $Mo_6Hal_{12}$ with Hal, which are identical or different, representing a halogen atom, and more particularly Hal represents Cl;
ii) molybdenum tetrahalides $(Hal')_4Mo$ with Hal', which are identical or different, corresponding to a halogen atom;
iii) molybdenum sulfur derivatives chosen from:

- molybdenum disulfides [*1317-33-5*], molybdenum(IV) sulfides, $MoS_2$;

- molybdenum sesquisulfide [*12033-33-9*]; dimolybdenum(III) trisulfides, $Mo_2S_3$;

iv) molybdenum acetate dimer [14221-06-8],

- the calcium salts are chosen from calcium hydroxides, calcium sulfates, calcium halides, calcium carboxylates, calcium phosphates, calcium borates and calcium carbonates.

**10.** Process according to any one of the preceding claims, in which the keratin fibres are mechanically wiped and/or dried and/or rinsed before carrying out the step comprising the application of the composition comprising the ingredients d) and e) as defined according to any one of Claims 1, 7 or 8.

**11.** Cosmetic composition for the dyeing of keratin fibres containing the compounds a), b), c), d), e) and f) as defined according to any one of Claims 1 to 9, and water or a mixture of water and of one or more organic solvents, it being understood that the pH of the composition is alkaline, i.e. greater than 7, and preferably between 8 and 12.

**12.** Use of one or more metal salt(s) as defined according to Claim 9 in a dyeing composition comprising one or more dihydroxyflavanol derivative(s) as defined according to any one of Claims 1 to 4 for improving the chromaticity of keratin fibres, in particular human keratin fibres such as the hair.

**Patentansprüche**

**1.** Verfahren zum Färben von Keratinfasern, insbesondere menschlichen Keratinfasern wie dem Haar, bei dem:

ein erster Schritt darin besteht, dass man auf die Keratinfasern eine Zusammensetzung aufbringt, die die folgenden Bestandteile umfasst:

a) ein oder mehrere Dihydroxyflavanol-Derivate, die durch Verbindungen der folgenden Formel (I) sowie Salze davon mit einer organischen oder anorganischen Säure, optische, geometrische und tautomere Isomere davon und Solvate davon wie die Hydrate wiedergegeben werden:

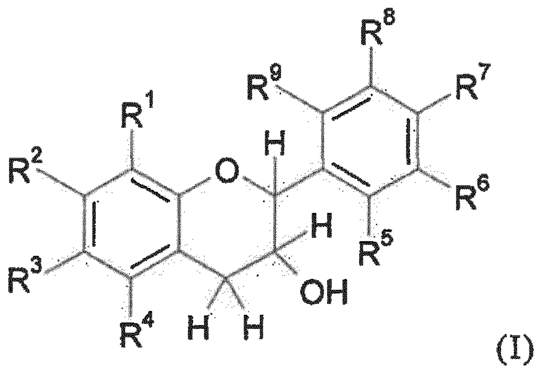

(I)

wobei:

• $R^1$, $R^2$, $R^3$, $R^4$, $R^5$, $R^6$, $R^7$, $R^8$ und $R^9$, die gleich oder verschieden sind, für:

- ein Wasserstoffatom,
- ein Halogenatom,
- eine Hydroxylgruppe,
- eine $(C_1\text{-}C_6)$ Alkylgruppe,
- eine $(C_1\text{-}C_6)$Alkoxygruppe,
- eine $(C_1\text{-}C_6)$Alkylthiogruppe,
- eine Carboxylgruppe,
- eine Alkoxycarbonylgruppe,
- eine gegebenenfalls substituierte Aminogruppe,
- eine gegebenenfalls substituierte lineare oder verzweigte Alkenylgruppe,

- eine gegebenenfalls substituierte Cycloalkylgruppe,
- eine Arylgruppe,
- eine substituierte Arylgruppe,
- eine Gruppe mit einem oder mehreren Siliciumatomen,
- eine (Di) ((hydroxy) ($C_1$-$C_6$) alkyl) aminogruppe,
- eine -O-Zuckergruppe, wie -O-Glucosid,
- eine R-Z-C(X)-Y- Gruppe, wobei R für ein Wasserstoffatom oder eine ($C_1$-$C_6$)Alkylgruppe steht, Y und Z, die gleich oder verschieden sein können, für eine Bindung, ein Sauerstoff- oder Schwefelatom oder eine -N(R')-Gruppe stehen, wobei R' für ein Wasserstoffatom oder eine ($C_1$-$C_6$)Alkylgruppe steht, Y gegebenenfalls auch für eine ($C_1$-$C_6$)Alkylengruppe stehen kann und X für ein Sauerstoff- oder Schwefelatom oder N-R'' steht, wobei R" für ein Wasserstoffatom oder eine ($C_1$-C6)Alkylgruppe steht,

stehen; wobei es sich versteht, dass mindestens zwei benachbarte Reste, die aus $R^1$, $R^2$, $R^3$, $R^4$, $R^5$, $R^6$, $R^7$, $R^8$ und $R^9$ ausgewählt sind, für eine Hydroxylgruppe stehen,

b) ein oder mehrere Mangansalze oder ein oder mehrere Zinksalze,
c) Wasserstoffperoxid oder ein oder mehrere Wasserstoffperoxid erzeugende Systeme und
f) ein oder mehrere Metallsalze, die aus Magnesiumsalzen, Molybdänsalzen und Calciumsalzen ausgewählt sind;

und dann ein zweiter Schritt darin besteht, dass man auf die Keratinfasern eine Zusammensetzung aufbringt, die die folgenden Bestandteile umfasst:

d) ein oder mehrere (Hydrogen) carbonate oder ein oder mehrere (Hydrogen)carbonate erzeugende Systeme, und
e) ein oder mehrere Alkalisierungsmittel, die von den Hydrogencarbonat(en) verschieden sind;

wobei es sich versteht, dass:

- mindestens eine der während des Färbeverfahrens verwendeten Zusammensetzungen einen alkalischen pH-Wert, d. h. einen pH-Wert von mehr als 7, aufweist und
- die Zusammensetzung, die den Bestandteil a) umfasst, wässrig ist.

**2.** Färbeverfahren nach Anspruch 1, **dadurch gekennzeichnet, dass** das Dihydroxyflavanol-Derivat bzw. die Dihydroxyflavanol-Derivate aus einem natürlichen Dihydroxyflavanol-Derivat bzw. natürlichen Dihydroxyflavanol-Derivaten ausgewählt ist bzw. sind.

**3.** Färbeverfahren nach einem der Ansprüche 1 oder 2, **dadurch gekennzeichnet, dass** das Dihydroxyflavanol-Derivat bzw. die Dihydroxyflavanol-Derivate aus

- Dihydroxyflavanolen wie Catechin und Epicatechingallat, deren Oligomeren und Polymeren, die als Proanthocyanidole bezeichnet werden, oder kondensierten Tanninen wie Theaflavin, Theaflavin-3'-O-gallat, Theaflavin-3,3'-O-digallat und Proanthocyanidinen A1, A2, B1, B2, B3 und C1
- und Mischungen der vorstehenden Verbindungen ausgewählt ist bzw. sind.

**4.** Färbeverfahren nach einem der Ansprüche 2 oder 3, **dadurch gekennzeichnet, dass** das natürliche Dihydroxyflavanol-Derivat bzw. die natürlichen Dihydroxyflavanol-Derivate aus Extrakten von Tieren, Bakterien, Pilzen, Algen, Pflanzen und Früchten ausgewählt ist bzw. sind.

**5.** Färbeverfahren nach einem der vorhergehenden Ansprüche, **dadurch gekennzeichnet, dass**:

- die Mangansalze aus:

i) Manganoxiden und
ii Halogeniden, Sulfaten, Phosphaten, Nitraten und Perchloraten, Carbonsäuresalzen, wie Gluconaten, sowie Mischungen davon
ausgewählt sind und

- die Zinksalze aus Zinksulfat, Zinkgluconat, Zinkchlorid, Zinklactat, Zinkacetat, Zinkglycinat und Zinkaspartat ausgewählt sind

und es sich spezieller bei den Salzen b) um Carbonsäuresalze von Mangan handelt.

**6.** Färbeverfahren nach einem der vorhergehenden Ansprüche, **dadurch gekennzeichnet, dass** es sich bei dem Bestandteil c) um Wasserstoffperoxid oder Harnstoffwasserstoffperoxid handelt.

**7.** Färbeverfahren einem der vorhergehenden Ansprüche, **dadurch gekennzeichnet, dass** es sich bei dem (Hydrogen)carbonat bzw. den (Hydrogen)carbonaten um Alkalimetall-, Erdalkalimetall- oder Ammonium(hydrogen)carbonat(e) handelt.

**8.** Färbeverfahren nach einem der vorhergehenden Ansprüche, **dadurch gekennzeichnet, dass** das Alkalisierungsmittel bzw. die Alkalisierungsmittel, das bzw. die von dem (Hydrogen)carbonat bzw. den(Hydrogen)carbonaten verschieden ist bzw. sind, aus:

- wässrigem Ammoniak,
- Alkanolaminen,
- oxyethylenierten Ethylendiaminen,
- oxypropylenierten Ethylendiaminen,
- anorganischen oder organischen Hydroxiden,
- Alkalimetallsilikaten,
- Aminosäuren und
- den Verbindungen der nachstehenden Formel (III):

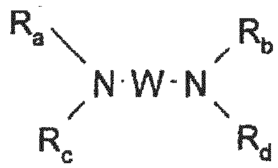
(III)

ausgewählt ist bzw. sind, wobei in der Formel (III) :

- W für eine zweiwertige ($C_1$-$C_8$) Alkylengruppe, vorzugsweise eine Propylengruppe, die gegebenenfalls insbesondere durch eine Hydroxylgruppe oder einen $C_1$-$C_4$-Alkylrest substituiert ist, steht;
- $R_a$, $R_b$, $R_c$ und $R_d$, die gleich oder verschieden sind, für ein Wasserstoffatom oder einen $C_1$-$C_4$-Alkyl- oder $C_1$-$C_4$-Hydroxyalkylrest stehen;

das Alkalisierungsmittel bzw. die Alkalisierungsmittel e) vorzugsweise aus Alkanolaminen, insbesondere Monoethanolamin, ausgewählt ist bzw. sind.

**9.** Färbeverfahren nach einem der vorhergehenden Ansprüche, **dadurch gekennzeichnet, dass**:

- die Magnesiumsalze aus Magnesiumhydroxiden, Magnesiumhalogeniden, Magnesiumsulfaten, Magnesiumnitraten, Carbonsäuresalzen von Magnesium und Magnesiumphosphaten ausgewählt sind,
- die Molybdänsalze aus:

i) binären Molybdänhalogeniden der Oxidationsstufen (II) bis (VI), die durch 6 Halogenatome von Mo(V), (IV) und (III) hexakoordiniert sind, wobei die Molybdänatome über Halogenbindungen gebunden sind, wie Mo (II) -Halogenid, das $[Mo_6Hal_8]^{4+}$-Cluster enthält, die an Halogenatome gebunden sind, was $Mo_6Hal_{12}$ ergibt, wobei die Variablen Hal, die gleich oder verschieden sind, für ein Halogenatom stehen und Hal spezieller für Cl steht;
ii) Molybdäntetrahalogeniden $(Hal')_4Mo$, wobei die Variablen Hal', die gleich oder verschieden sind, einem Halogenatom entsprechen;
iii) Molybdänschwefelderivaten, die aus:

- Molybdändisulfiden [*1317-33-5*], Molybdän(IV)-sulfiden, $MoS_2$;

- Molybdänsesquisulfid [*12033-33-9*]; Dimolybdän-(III)-trisulfiden, $Mo_2S_3$;
ausgewählt sind;

iv) Molybdänacetat-Dimer [14221-06-8] ausgewählt sind,

- die Calciumsalze aus Calciumhydroxiden, Calciumsulfaten, Calciumhalogeniden, Calciumcarboxylaten, Calciumphosphaten, Calciumboraten und Calciumcarbonaten ausgewählt sind.

**10.** Verfahren nach einem der vorhergehenden Ansprüche, bei dem die Keratinfasern vor der Durchführung des Schritts, der das Aufbringen der Zusammensetzung, die die Bestandteile d) und e) gemäß einem der Ansprüche 1, 7 oder 8 umfasst, umfasst, mechanisch abgewischt und/oder getrocknet und/oder gespült werden.

**11.** Kosmetische Zusammensetzung zum Färben von Keratinfasern, enthaltend die Verbindungen a), b), c), d), e) und f) gemäß einem der Ansprüche 1 bis 9 und Wasser oder eine Mischung von Wasser und einem oder mehreren organischen Lösungsmitteln, wobei es sich versteht, dass der pH-Wert der Zusammensetzung alkalisch, d. h. größer als 7, ist und vorzugsweise zwischen 8 und 12 liegt.

**12.** Verwendung eines oder mehrerer Metallsalze gemäß Anspruch 9 in einer Färbezusammensetzung, die ein oder mehrere Dihydroxyflavanol-Derivate gemäß einem der Ansprüche 1 bis 4 umfasst, zur Verbesserung der Buntheit von Keratinfasern, insbesondere menschlichen Keratinfasern wie dem Haar.

**Revendications**

**1.** Procédé de coloration des fibres kératiniques, en particulier des fibres kératiniques humaines telles que les cheveux, dans lequel ; une première étape consiste à appliquer sur lesdites fibres kératiniques une composition comprenant les ingrédients suivants :

a) un ou plusieurs dérivé(s) de dihydroxyflavanol(s) représentés par les composés de formule (I) suivante ainsi que leurs sels d'acide organique ou inorganique, leurs isomères optiques, géométriques, tautomères et leurs solvates tels que les hydrates :

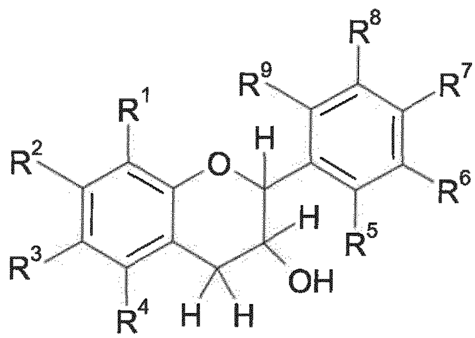

(I)

dans laquelle :

• $R^1$, $R^2$, $R^3$, $R^4$, $R^5$, $R^6$, $R^7$, $R^8$ et $R^9$, identiques ou différents, représentent :

- un atome d'hydrogène,
- un atome d'halogène
- un groupe hydroxy,
- un groupe ($C_1$-$C_6$)alkyle,
- un groupe ($C_1$-$C_6$)alcoxy
- un groupe ($C_1$-$C_6$)alkylthio,
- un groupe carboxyle,
- un groupe alcoxycarbonyle,
- un groupe amino éventuellement substitué,

- un groupe alcényle linéaire ou ramifié éventuellement substitué,
- un groupe cycloalkyle éventuellement substitué,
- un groupe aryle,
- un groupe aryle substitué,
- un groupe contenant un ou plusieurs atomes de silicium,
- un groupe (di)((hydroxy)($C_1$-$C_6$)alkyl)amino,
- un groupe -O-sucre tel que -O-glucoside,
- un groupe R-Z-C(X)-Y- avec R représentant un atome d'hydrogène, ou un groupe ($C_1$-$C_6$)alkyle, Y et Z, identiques ou différents, représentent une liaison, un atome d'oxygène ou de soufre ou un groupe -N(R')- avec R' représentant un atome d'hydrogène ou un groupe ($C_1$-$C_6$)alkyle, Y pouvant également représenter un groupe ($C_1$-$C_6$)alkylène, et X représentant un atome d'oxygène ou de soufre, ou N-R" avec R" représentant un atome d'hydrogène ou un groupe ($C_1$-$C_6$)alkyle ;

étant entendu qu'au moins deux radicaux adjacents choisis parmi $R^1$, $R^2$, $R^3$, $R^4$, $R^5$, $R^6$, $R^7$, $R^8$ et $R^9$, représentent un groupement hydroxy,

b) un ou plusieurs sel(s) de manganèse ou un ou plusieurs sel(s) de zinc,
c) du peroxyde d'hydrogène ou un ou plusieurs système(s) générateur(s) de peroxyde d'hydrogène, et
f) un ou plusieurs sel(s) métallique(s) choisi(s) parmi les sels de magnésium, les sels de molybdène et les sels de calcium ;

puis, une seconde étape consiste à appliquer sur lesdites fibres kératiniques une composition comprenant les ingrédients suivants

d) un ou plusieurs (bi)carbonate(s) ou un ou plusieurs système(s) générateur(s) de (bi)carbonate(s), et
e) un ou plusieurs agent(s) alcalinisant(s) différent(s) du ou de(s) bicarbonate(s) ;

étant entendu que :

- au moins une des compositions utilisées durant ledit procédé de coloration a un pH alcalin, soit supérieur à 7 ; et
- la composition comprenant l'ingrédient a) est aqueuse.

**2.** Procédé de coloration selon la revendication 1, **caractérisé en ce que** le ou les dérivé(s) de dihydroxyflavanol(s) sont choisis parmi les dérivé(s) de dihydroxyflavanol(s) naturel(s).

**3.** Procédé de coloration selon la revendication 1 ou 2, **caractérisé en ce que** le ou les dérivé(s) de dihydroxyflavanol(s) sont choisis parmi :

- les dihydroxyflavonols comme la catéchine et le gallate d'épichatéchine, leurs oligomères et polymères appelés proanthocyanidols ou tanins condensés comme la théaflavine, la théaflavine 3'-O-gallate, la théaflavine 3,3'-O-digallate, les proanthocyanidines A1, A2, B1, B2, B3 et C1 ;
- et les mélanges des composés précédents.

**4.** Procédé de coloration selon l'une quelconque des revendications 2 ou 3, **caractérisé en ce que** le ou les dérivé(s) de dihydroxyflavanol(s) naturel(s) sont choisis parmi les extraits d'animaux, de bactéries, de champignons, d'algues, de plantes et de fruits.

**5.** Procédé de coloration selon l'une quelconque des revendications précédentes, **caractérisé en ce que** :

- les sels de manganèse sont choisis parmi :

  i) les oxydes de manganèse et
  ii) les halogénures, les sulfates, phosphates, nitrates et perchlorates, les sels d'acides carboxyliques tels que les gluconates ainsi que leurs mélanges, et

- les sels de zinc sont choisis parmi le sulfate de zinc, le gluconate de zinc, le chlorure de zinc, le lactate de zinc, l'acétate de zinc, le glycinate de zinc et l'aspartate de zinc ;

et plus particulièrement les sels b) sont des sels d'acide carboxyliques de manganèse.

**6.** Procédé de coloration selon l'une quelconque des revendications précédentes, **caractérisé en ce que** l'ingrédient c) est du peroxyde d'hydrogène ou du peroxyde d'urée.

**7.** Procédé de coloration selon l'une quelconque des revendications précédentes, **caractérisé en ce que** le ou les (bi)carbonates sont des (bi)carbonates alcalins, alcalino-terreux ou d'ammonium.

**8.** Procédé de coloration selon l'une quelconque des revendications précédentes, **caractérisé en ce que** le ou les agents alcalins différents du ou de(s) bicarbonate(s) sont choisis parmi :

- l'ammoniaque,
- les alcanolamines,
- les éthylènediamines oxyéthylénées
- les éthylènediamines oxypropylénées,
- les hydroxydes minéraux ou organiques,
- les silicates de métaux alcalins,
- les acides aminés, et
- les composés de formule (II) suivante :

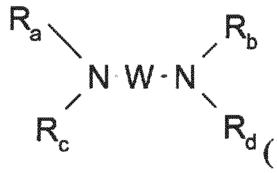
(II)

formule (II) dans laquelle :

- W est un groupe divalent ($C_1$-$C_8$)alkylène, de préférence propylène, éventuellement substitué notamment par un groupement hydroxy ou un radical alkyle en $C_1$-$C_4$ ;
- $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$ ;

de préférence, le ou les agents alcalins e) sont choisis parmi les alcanolamines, en particulier la monoéthanolamine.

**9.** Procédé de coloration selon l'une quelconque des revendications précédentes, **caractérisé en ce que** :

- les sels de magnésium sont choisis parmi les hydroxydes de magnésium, les halogénures de magnésium, les sulfates de magnésium, les nitrates de magnésium, les sels d'acide carboxylique de magnésium et les phosphates de magnésium,
- les sels de molybdène sont choisis parmi :

  i) les halogénures de molybdène binaires d'états d'oxydation (II) à (VI), hexacoordiné par 6 halogènes de Mo(V), (IV) et (III), les atomes de molybdène étant liés par des liaisons halogènes telles que l'halogénure de Mo(II) contenant des clusters $[Mo_6Hal_8]^{4+}$ liés à des atomes d'halogène pour donner $Mo_6Hal_{12}$ avec Hal, identiques ou différents, représentant un atome d'halogène, et plus particulièrement Hal représente Cl ;
  ii) les tétrahalogénures de molybdène $(Hal')_4Mo$ avec Hal', identiques ou différents, avec Hal' correspondant à un atome d'halogène,
  iii) les dérivés soufrés du molybdène choisis parmi :

- les disulfures de Molybdenum [*1317-33-5*], sulfures de molybdenum(IV), $MoS_2$
- les sesquisulfure de Mo [*12033-33-9*] ; les trisulfures de dimolybdène (III), $Mo_2S_3$ ;

  iv) l'acétate de molybdène dimérique [14221-06-8],

- les sels de calcium sont choisis parmi les hydroxydes de calcium, les sulfates de calcium, les halogénures de calcium, les carboxylates de calcium, les phosphates de calcium, les borates de calcium et les carbonates de calcium.

**10.** Procédé selon l'une quelconque des revendications précédentes, dans lequel un essuyage mécanique et/ou un séchage et/ou un rinçage des fibres kératiniques est effectué avant de réaliser l'étape comprenant l'application de la composition comprenant les ingrédients d) et e) tels que définis selon l'une quelconque des revendications 1, 7 ou 8.

**11.** Composition cosmétique pour la coloration des fibres kératiniques contenant les composés a), b), c), d), e) et f) tels que définis selon l'une quelconque des revendications 1 à 9, et de l'eau ou un mélange d'eau et d'un ou plusieurs solvants organiques, étant entendu que le pH de la composition est alcalin soit supérieur à 7, et de préférence compris entre 8 et 12.

**12.** Utilisation d'un ou plusieurs sels métalliques tels que définis selon la revendication 9 dans une composition de coloration comprenant un ou plusieurs dérivé(s) de dihydroxyflavanol(s) tels que définis selon l'une quelconque des revendications 1 à 4, pour améliorer la chromaticité des fibres kératiniques en particulier des fibres kératiniques humaines telles que les cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2196180 A
- EP 2196181 A
- FR 2814943
- US 5008093 A
- US 3376110 A
- US 5183901 A

### Non-patent literature cited in the description

- **METAL ; DYES.** *Ullmann's Encyclopedia,* 2005, vol. 5.1, 8
- Kirk-Othmer's Encyclopedia of Chemical Technology Copyright ©. John Wiley & Sons Inc, 17 September 2009
- **EDWARD I. STIEFFEL.** *Molybdenum compounds,* 871-895
- Molybdenum and Molybdenum Compounds. Ullmann's Encyclopedia. WILEY-VCH Verlag GmbH & Co, 2000